# EUROPEAN PATENT APPLICATION

(11) **EP 4 494 670 A1**
(43) Date of publication of application: **22.01.2025**
(21) Application number: 24188819.7
(22) Date of filing: 16.07.2024
(51) Int. Cl.: A61M 1/36, A61M 5/178, A61M 5/36

(54) **PURGE SYRINGE**

(30) Priority: 20.07.2023 US 202318224498
(71) Applicant: CardiacAssist, Inc., Pittsburgh, PA 15238 (US)
(72) Inventor: HARTOGS, Ruben, Pittsburgh, PA 15232 (US)
(74) Representative: Buzzi, Notaro & Antonielli d'Oulx S.p.A.

(57) **Abstract**

A syringe for de-airing a liquid conduit of an extracorporeal life support system includes an inner barrel having an elongated distal tip defining a distal tip opening, and a proximal open end, and an outer barrel at least partially surrounding the inner barrel. An inner wall of the outer barrel is spaced apart from at least a portion of an outer wall of the inner barrel defining a space therebetween. The outer barrel has a distal end with at least one opening proximal of the distal tip opening of the inner barrel, and a proximal opening such that the space is open to the ambient environment via the proximal opening. The syringe also includes a plunger disposed within and sealing the proximal open end of the inner barrel with a friction fit with an inner wall of the inner barrel.

## Description

### TECHNICAL FIELD

The present disclosure pertains to a syringe for use in a liquid conduit of an extracorporeal life support (ECLS) system. More particularly, the present disclosure pertains to a purge syringe with multiple chambers for removing air from fluid connectors.

### BACKGROUND

Some medical procedures (e.g., medical procedures which treat cardiac or respiratory disease) may require the use of a life support system that supports cardiac and pulmonary functions by artificially supporting the heart and the lung function. In some instances, this may be carried out by an extracorporeal membrane oxygenation (ECMO) system, also known as an extracorporeal life support (ECLS) system. ECLS is an extracorporeal system which provides both cardiac and respiratory support to a patient whose heart and lungs are unable to provide an adequate amount of gas exchange to sustain life. ECLS works by removing blood from a patient's body to purify and oxygenate the red blood cells while also removing carbon dioxide. The purified and oxygenated blood is then returned to the patient.

ECLS systems may include multiple devices that together form a blood recirculation loop between the patient and a blood oxygenator. For example, some ECLS systems may include a blood reservoir, a blood pump to power blood flow, an oxygenator to oxygenate the blood, a device to filter the blood (which may be included within the oxygenator in some systems), a heat exchanger (to heat and/or cool blood), one or more oxygen sensors positioned at various locations along blood pathways and a control console. It can be appreciated that a blood pathway (e.g., cannula, tubing) may extend from the patient to the blood reservoir, then towards a blood pump, then pass through the oxygenator and close the loop by returning to the patient. Accordingly, the blood pump may assist the heart by pumping blood through the circulation loop, while the oxygenator may assist the lungs by oxygenating blood that is eventually returned to the patient.

During initiation of the ECLS, all air in the fluid conduit line or tubing must be removed, generally by flushing the conduit with saline. When connecting two sections of conduit, a small amount of air may remain in the connector. Accordingly, a device and method for removing all air from the conduit and connector is needed.

### BRIEF SUMMARY

This disclosure provides design, material, manufacturing method, and use alternatives for medical devices, including elements of an ECLS system.

One example is a syringe for de-airing a liquid conduit of an extracorporeal life support system. The syringe includes an inner barrel having an elongated distal tip defining a distal tip opening, and an outer barrel at least partially surrounding the inner barrel. An inner wall of the outer barrel is spaced apart from at least a portion of an outer wall of the inner barrel defining a space therebetween. The outer barrel has a distal end with at least one opening, and a proximal opening. The at least one opening is proximal of the distal tip opening of the inner barrel. A plunger is disposed within the inner barrel and axially movable relative to the inner barrel. The plunger has a friction fit with an inner wall of the inner barrel.

Alternatively or additionally to any of the examples above, in another example, the inner barrel and the outer barrel are coaxial.

Alternatively or additionally to any of the examples above, in another example, one or more radially extending supports connect the outer wall of the inner barrel to the inner wall of the outer barrel.

Alternatively or additionally to any of the examples above, in another example, the at least one opening in the outer barrel is a distally facing opening.

Alternatively or additionally to any of the examples above, in another example, the distally facing opening surrounds the elongated distal tip of the inner barrel.

Alternatively or additionally to any of the examples above, in another example, the distal tip tapers distally to the distal tip opening.

Alternatively or additionally to any of the examples above, in another example, the outer barrel includes a coupler for coupling with a connector in the liquid conduit of the extracorporeal life support system.

Alternatively or additionally to any of the examples above, in another example, the coupler includes a distal tubular extension with inner threading, wherein the elongated distal tip of the inner barrel extends through the distal tubular extension and the distal tip opening is spaced apart distally from a distal end of the distal tubular extension.

Alternatively or additionally to any of the examples above, in another example, the distal tubular extension forms a luer lock coupling with the connector in the liquid conduit of the extracorporeal life support system.

Alternatively or additionally to any of the examples above, in another example, the space is open to an ambient environment via the proximal opening.

Alternatively or additionally to any of the examples above, in another example, a portion of the outer wall of the inner barrel is fixed to a portion of the inner wall of the outer barrel.

Alternatively or additionally to any of the examples above, in another example, the distal tip opening is offset from a central longitudinal axis of the inner barrel and centered on a central longitudinal axis of the outer barrel.

Alternatively or additionally to any of the examples above, in another example, the inner barrel is filled with saline and sealed with the plunger.

Another example is a syringe for de-airing a conduit of an extracorporeal life support system. The syringe includes an inner barrel having an elongated distal tip defining a distal tip opening, a plunger disposed within the inner barrel and axially movable relative to the inner barrel, and an outer barrel coupled to and surrounding the inner barrel with an inner wall of the outer barrel spaced apart from at least a portion an outer wall of the inner barrel and defining a space therebetween. The outer barrel has a proximal opening and a distal end defining a distally facing opening. The distal end includes a coupler. The elongated distal tip of the inner barrel extends through the distally facing opening with the distal tip opening spaced apart distal of the distally facing opening of the outer barrel. The distally facing opening is in fluid communication with the space between the inner barrel and the outer barrel.

Alternatively or additionally to any of the examples above, in another example, the inner barrel and the outer barrel are coaxial.

Alternatively or additionally to any of the examples above, in another example, the elongated distal tip tapers distally to the distal tip opening.

Alternatively or additionally to any of the examples above, in another example, the coupler includes a distal extension with internal threading.

Alternatively or additionally to any of the examples above, in another example, a portion of the outer wall of the inner barrel is fixed to a portion of the inner wall of the outer barrel.

Alternatively or additionally to any of the examples above, in another example, the space is open to an ambient environment via the proximal opening.

Another example is a method of de-airing a liquid conduit of an extracorporeal life support system. The method includes coupling a de-airing syringe with a connector of the liquid conduit. The de-airing syringe includes an inner barrel having a distal tip with a distal tip opening, a plunger disposed within the inner barrel and axially movable relative to the inner barrel, and an outer barrel at least partially surrounding the inner barrel. An inner wall of the outer barrel is spaced apart from at least a portion an outer wall of the inner barrel defining a space therebetween. The outer barrel has a distal end with at least one distally facing opening and a proximal opening. The at least one distally facing opening is proximal of the distal tip opening of the inner barrel. The outer barrel includes a coupler configured to couple with the connector of the liquid conduit. The inner barrel contains saline and the space between the inner barrel and the outer barrel is open to an ambient environment via the proximal opening. The connector contains saline. Coupling the de-airing syringe with the connector includes inserting the distal tip transversely into a side lumen of the connector such that the distal tip opening is under a liquid line within a main lumen of the connector. Thereafter, the method includes depressing the plunger to inject the saline inside the inner barrel into the main lumen of the connector thereby forcing air within an upper region of the main lumen out through the distally facing opening and into the space between the inner and outer barrels.

The above summary of some embodiments is not intended to describe each disclosed embodiment or every implementation of the present disclosure. The Figures, and Detailed Description, which follow, more particularly exemplify some of these embodiments.

### BRIEF DESCRIPTION OF THE DRAWINGS

The disclosure may be more completely understood in consideration of the following detailed description in connection with the accompanying drawings, in which:
FIG. 1 is a side view of an exemplary connector;
FIG. 2 is a cross-sectional view of an exemplary syringe;
FIG. 3 is a top down view of the syringe of FIG. 2 with the plunger removed;
FIG. 4 is a cross-sectional view of the syringe of FIG. 2 coupled to a connector;
FIG. 5 is a partial cross-sectional view of another exemplary syringe coupled to another type of connector;
FIG. 6 is a cross-sectional view of a further exemplary syringe; and
FIG. 7 is a top down view of the syringe of FIG. 6 with the plunger removed.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the spirit and scope of the disclosure.

### DETAILED DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar structures in different drawings are numbered the same. The drawings, which are not necessarily to scale, depict illustrative embodiments and are not intended to limit the scope of the disclosure.

In a normal heart, blood circulates via a closed path whereby deoxygenated (venous) blood enters the right atrium via both the superior vena cava and inferior vena cava. The venous blood then passes through the right ventricle and is pumped via the pulmonary artery to the lungs, where it absorbs oxygen. After absorbing oxygen in the lungs, the blood becomes oxygenated arterial blood. The oxygenated arterial blood is then returned via the pulmonary veins to the left atrium and is passed to the left ventricle. The oxygenated arterial blood is then pumped through the aorta and eventually throughout the body.

It can be appreciated that if the lungs are incapable of sufficiently oxygenating blood, an oxygenator located outside the body may be used to oxygenate the blood. As discussed above, extracorporeal life support (ECLS) is a breathing and heart pumping life support system that may be utilized to support patients while medical treatments are performed to treat their underlying illness. When supported via an ECLS system, oxygenation of the patient's blood and removal of carbon dioxide may occur outside the body.

ECLS is generally performed using a heart-lung bypass system, which may be referred to as a "circuit." The circuit may include one or more tubing pathways designed to transfer blood from a patient's body to the oxygenator and back into the patient. As described above, the oxygenator may add oxygen to the blood while also removing carbon dioxide (e.g., the oxygenator performs the function of a healthy lung).

In some examples, an ECLS circuit may include a blood pump, oxygenator, tubing pathways (for transfer to and from the body), flow and/or pressure sensors, a heat exchanger (to cool and/or heat the blood), a computing console, and arterial and/or venous access points for the collection of blood in the circuit. During the setup of the circuit, a wet-to-wet connection must be made between saline-filled tubing connected to the blood pump and oxygenator and blood-filled tubing coming from arterial and/or venous access points in the patient.

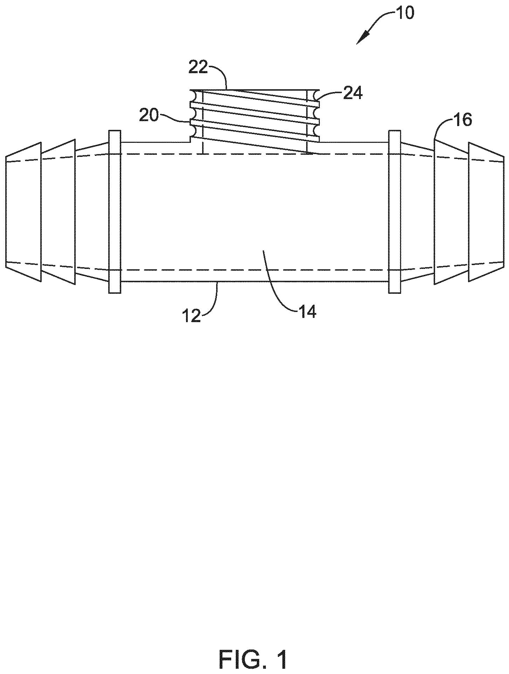

FIG. 1 illustrates an example connector 10 that may be used to connect tubing connected to the ECLS system and tubing connected to arterial and/or venous access points in the patient. The connector 10 may include a main body 12 with a first end, a second end, and a main lumen 14 extending longitudinally therebetween. The first and second ends may have the same diameter, or they may be different. For example, the first end may be 3/8 inch and the second end may be ½ inch. One or more projections 16 configured to provide a friction fit with tubing may be disposed adjacent the first and second ends. The projections 16 are shown as barb fittings configured to be inserted into the lumen of a tubular member (e.g., tubing). The barb fittings may include one or more projecting annular ridges configured to form an interface fit against an inner surface of a tubular member (e.g., tubing). The barb fittings may be any desired size to fit into any desired size of medical tubing. For instance, the barb fittings may be 3/8-inch diameter barb fittings configured to be inserted into a 3/8-inch diameter medical tubing. In other instances, the barb fittings may be of a different size, such as 1/4-inch diameter, 5/16-inch diameter, 1/2-inch diameter, or other desired size to accommodate a corresponding size of medical tubing. The connector 10 may have a transverse extension 20 defining a side lumen 22 in fluid communication with the main lumen 14. The transverse extension 20 may have a length of about 0.25 inch to about 1 inch. In some examples, the transverse extension 20 may have a length of about 0.45 inch. The transverse extension 20 may include external threading 24 configured to engage a device such as a syringe in a fluid tight connection. In other examples, the transverse extension 20 may have a smooth inner and outer surface to provide a friction fit with a device or syringe.

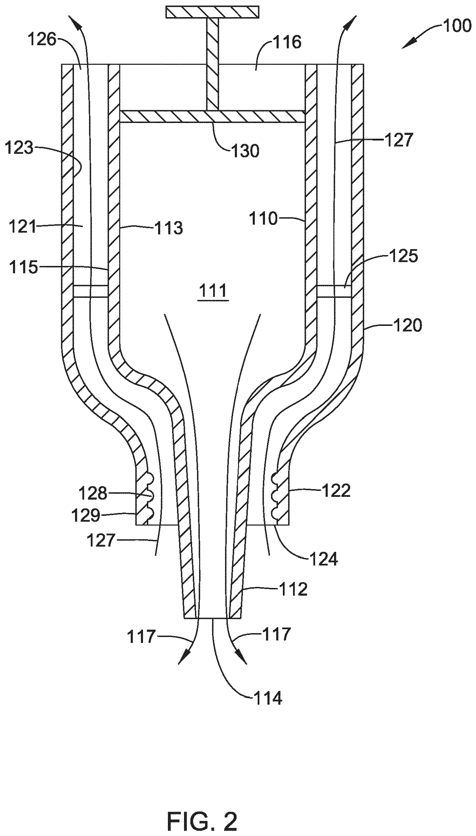

An example multi-chambered purge syringe 100 for de-airing (e.g., purging from) a liquid conduit such as those in an ECLS is illustrated in FIG. 2. The syringe 100 may include an inner barrel 110 defining a chamber 111 therein. The inner barrel 110 may have an elongated distal tip 112 defining a distal tip opening 114 in fluid communication with the chamber 111 for expelling fluid therefrom. The elongated distal tip 112 may taper down distally to the distal tip opening 114. The inner barrel 110 may have a proximal open end 116 configured to receive a plunger 130 disposed within and sealing the proximal open end 116. The chamber 111 of the inner barrel 110 may be filled with liquid such as saline, and sealed with the plunger 130, such that the inner barrel 110 is devoid of air.

The plunger 130 may form a friction fit with an inner wall 113 of the inner barrel 110. An outer barrel 120 may at least partially surround the inner barrel 110, with an inner wall 123 of the outer barrel 120 spaced apart from at least a portion of an outer wall 115 of the inner barrel 110 defining a space 121 therebetween. In some instances, the space 121 may be an annular space between the outer wall 115 of the inner barrel 110 and the inner wall 123 of the outer barrel 120. The outer barrel 120 may have a distal end 122 with at least one opening 124, and a proximal opening 126. In some instances the proximal opening 126 may be at the proximal end of the outer barrel 120. In other instances, the proximal opening 126 may extend through a sidewall of the outer barrel 120. The opening 124 is proximal of the distal tip opening 114 of the inner barrel 110, and the opening 124 may be distally facing. Thus, the distal tip 112 of the inner barrel 110 may extend distal of the opening 124 of the outer barrel 120. In some examples, the distally facing opening 124 may completely surround the distal tip 112 of the inner barrel 110. The distal tip 112 may be spaced apart from the inner surface of the distal end 122 such that the opening 124 is in fluid communication with the space 121 between the inner barrel 110 and the outer barrel 120.

The distal end 122 of the outer barrel 120 may define a distal tubular extension 129 with an engagement member or coupling 128 for coupling with a port of a connector, such as the connector 10, in the ECLS system. The connector 10 may join two sections of liquid conduit or tubing (not shown) of the ECLS system.

In the example shown in FIG. 2, the engagement member or coupling 128 includes internal threading 128 on the distal tubular extension 129. In other examples, the engagement member or coupling 128 may be a smooth surface configured to provide a friction fit with the connector. In yet other embodiments, the engagement member or coupling 128 may be otherwise configured to engagement with or couple to a port of the connector. The distal tip 112 of the inner barrel 110 may extend through the distal tubular extension 129 and the distal tip opening 114 may be spaced apart distally from the distal end 122 of the distal tubular extension 129.

This configuration of the distal tip 112 of the inner barrel 110 extending distally through the opening 124 in the outer barrel 120 and being spaced apart from the outer barrel 120 allows for liquid, such as saline, disposed within the chamber 111 to be expressed (indicated by arrows 117) at a location distal of the opening 124 of the outer barrel 120, while fluid such as air or liquid may be pushed proximally through the opening 124 and into the space 121 between the inner barrel 110 and the outer barrel 120, as indicated by arrows 127 in FIG. 2.

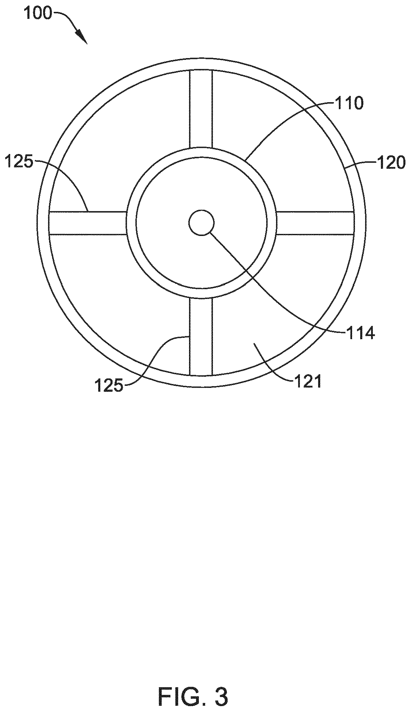

In some examples, the inner barrel 110 and the outer barrel 120 may be coaxial and may be coupled by one or more radially extending supports 125 connecting the outer wall 115 of the inner barrel 110 to the inner wall 123 of the outer barrel 120. See FIGS. 2 and 3. The supports 125 may be spaced proximal of the at least one opening 124. In the example shown in FIG. 3, the syringe 100 has four supports 125 spaced equally around the inner barrel 110 and spanning the space 121 between the outer wall 115 of the inner barrel 110 and the inner wall 123 of the outer barrel 120. In other examples, one, two, three, or more than four supports 125 may secure the inner and outer barrels together.

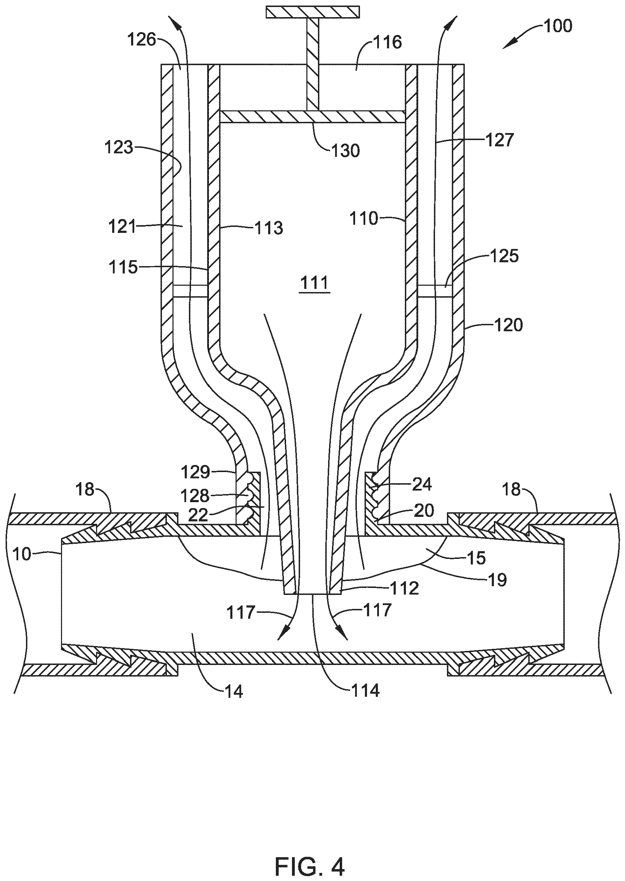

FIG. 4 shows the syringe 100 of FIG. 2 coupled with the port of the connector 10 of FIG. 1. The connector 10 may be connected to two sections of conduit or tubing 18 in the ECLS system. The distal tubular extension 129 may have a length and diameter configured to engage the transverse extension 20 forming the port of the connector 10, which may be sized as described above. The distal tip 112 of the syringe 100 is inserted into the side lumen 22 of the transverse extension 20 of the connector 10. The internal threading 128 on the distal tubular extension 129 is threaded onto the external threading on the transverse extension 20 to form a fluid tight seal between the syringe 100 and the connector 10. The threaded connection may form a luer lock coupling between the syringe 100 and the ECLS system. In other instances, the distal tubular extension 129 of the outer barrel 120 of the syringe 100 may otherwise be coupled to the transverse extension 20 of the connector 10. When coupled to the transverse extension 20 of the connector 10, both the inner chamber 111 and the space 121 may be in fluid communication with the main lumen 14 of the connector 10. In some examples, the chamber 111 of the inner barrel 110 may have a fluid volume of about 20 ml to about 100 ml. In one example, the inner barrel 110 has a fluid volume of about 50 ml.

When the syringe 100 is coupled to a connector 10 filled with liquid, the distal tip opening 114 is positioned under the liquid line 19 so as to be in fluid communication with the liquid, such as saline, within the main lumen 14 of the connector 10. After the syringe 100 is coupled to the connector 10, a pocket of air 15 may be present at the top of the main lumen 14 adjacent the opening to the distal tubular extension 129. The inner barrel 110 may be pre-filled with liquid such as saline, and sealed with the plunger 130, such that the chamber 111 is devoid of air. With the distal tip opening 114 positioned under the liquid line 19, when the liquid inside the inner barrel 110 is discharged and injected through the distal tip opening 114, indicated by arrows 117, the air 15 is forced up through the side lumen 22 of the connector exterior of the distal tip 112 and into the space 121 between the inner barrel 110 and outer barrel 120 of the syringe 100, indicated by arrows 127. In some instances the upper or proximal end of the space 121 may be open to the ambient environment (e.g., atmosphere), or otherwise allow the air 15 to escape from the connector 10 through the lumen 22 of the transverse extension 20 while the syringe 100, namely the distal tubular extension 129 of the outer barrel 120, is sealingly coupled to the transverse extension 20 of the connector 10. For example, the space 121 may be open to an ambient environment via the proximal opening 126.

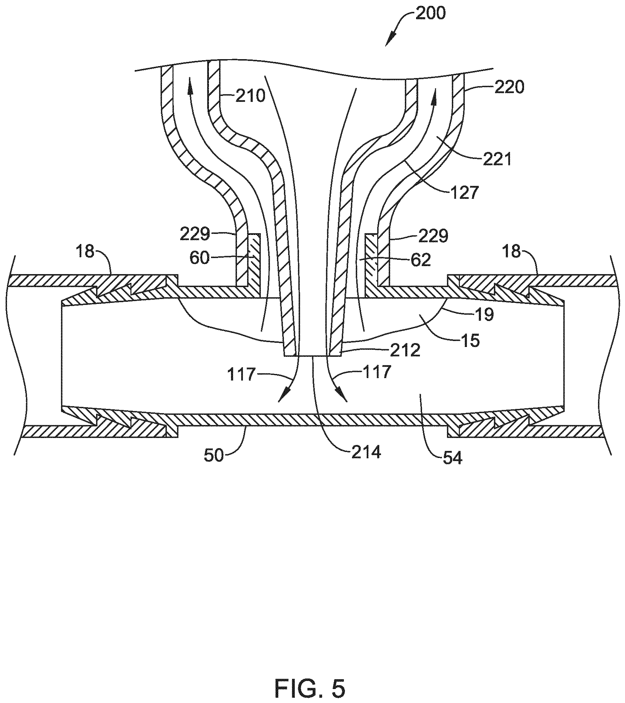

A friction fit engagement between a de-airing syringe 200 and connector 50 is illustrated in FIG. 5. The syringe 200 has an inner barrel 210 and outer barrel 220 with the same structure as the syringe 100 described except the distal tubular extension 229 may be devoid of threading, and be otherwise configured to sealing connect to the transverse extension 60 forming a side port of the connector 50. Similar to the syringe 100 above, the syringe 200 may include an inner barrel 210 defining a chamber 211 therein. The inner barrel 210 may have an elongated distal tip 212 defining a distal tip opening 214 in fluid communication with the chamber 211 for expelling fluid therefrom. The elongated distal tip 212 may taper down distally to the distal tip opening 214. The inner barrel 210 may receive a plunger (not shown) disposed within the chamber 211 that may be actuated to expel fluid from the distal tip opening 214. The chamber 211 of the inner barrel 210 may be filled with liquid such as saline such that the inner barrel 210 is devoid of air.

The outer barrel 220 may at least partially surround the inner barrel 210, with an inner wall of the outer barrel 220 spaced apart from at least a portion an outer wall of the inner barrel 210 defining a space 221 therebetween. In some instances, the space 221 may be an annular space between the outer wall of the inner barrel 210 and the inner wall of the outer barrel 220. In some instances, the inner barrel 210 and the outer barrel 220 may be coaxial. A distal opening, which may be a distally facing opening, of the outer barrel 220 is proximal of the distal tip opening 214 of the inner barrel 210. Thus, the distal tip 212 of the inner barrel 210 may extend distal of the distal opening of the outer barrel 220. In some examples, the distally facing opening of the outer barrel 220 may completely surround the distal tip 212 of the inner barrel 210. The distal tip 212 may be spaced apart from the inner surface of the distal end of the outer barrel 220 such that the distal opening of the outer barrel 220 is in fluid communication with the space 221 between the inner barrel 210 and the outer barrel 220.

The distal end of the outer barrel 220 may define a distal tubular extension 229 configured for coupling with a port of a connector, such as the transverse extension 60 of the connector 50, in the ECLS system. The connector 50 may join two sections of liquid conduit or tubing 18 of the ECLS system.

The connector 50 may be similar to the connector 10 except the transverse extension 60 may be devoid of external threading. When the syringe distal tip 212 is inserted through the side lumen 62 and into the main lumen 54 of the connector 50, the distal tubular extension 229 may engage the transverse extension 60 with a friction fit to provide a fluid tight seal therebetween. In some instances the distal tubular extension 229 may sealingly surround the transverse extension 60, as shown in FIG. 5. In other instances, the distal tubular extension 229 of the outer barrel 220 may be inserted into the transverse extension 60 such that the transverse extension 60 sealingly surrounds the distal tubular extension 229 of the outer barrel 220. When the syringe 200 is coupled to the connector 50, the distal tip opening 214 is positioned under the liquid line 19 so as to be in fluid communication with the liquid, such as saline, within the main lumen 54 of the connector 50. After the syringe 200 is coupled to the connector 50, a pocket of air 15 may be present at the top of the main lumen 54 adjacent the distal end of the distal tubular extension 229. The inner barrel 210 may be filled with liquid such as saline, and sealed with the plunger, such that the inner barrel 210 is devoid of air. With the distal tip opening 214 positioned under the fluid line, when the liquid inside the inner barrel 210 is expelled and injected through the distal tip opening 214, indicated by arrows 117, the air 15 is forced up through the side lumen 62 of the transverse extension 60 of the connector 50 and into the space 221 between the inner barrel 210 and outer barrel 220 of the syringe 200, indicated by arrows 127. In some instances the upper or proximal end of the space 221 may be open to the ambient environment (e.g., atmosphere), or otherwise allow the air 15 to escape from the connector 50 through the lumen 62 of the transverse extension 60 while the syringe 200, namely the distal tubular extension 229 of the outer barrel 220, is sealingly coupled to the transverse extension 60 of the connector 50. For example, the space 221 may be open to an ambient environment via a proximal opening of the outer barrel 220.

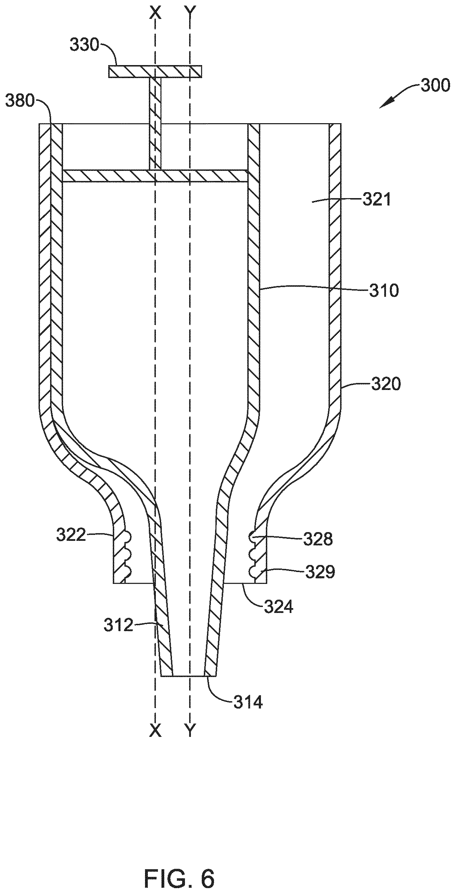

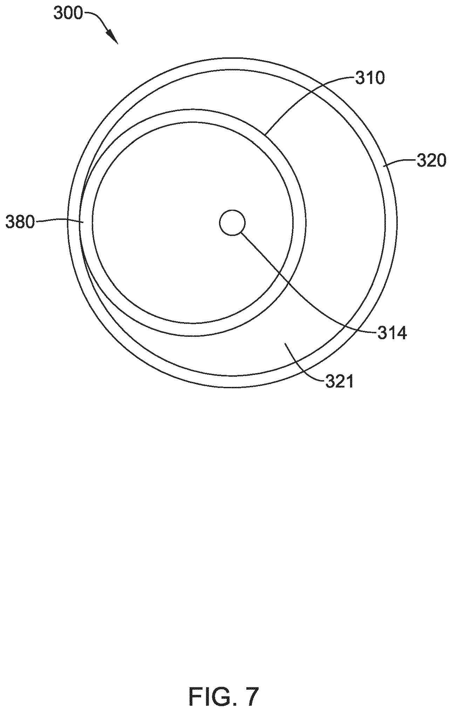

In another example, shown in FIGS. 6 and 7, the de-airing syringe 300 may be configured such that the inner barrel 310 may not be coaxial with the outer barrel 320. For example, the de-airing syringe 300 may have a portion of the outer surface of the inner barrel 310 fixed to a portion of the inner surface of the outer barrel 320 at junction 380, such as a bonding region. See FIGS. 6 and 7. In some examples, the junction or bonding region 380 may extend axially along an interface between the inner and outer barrels, as shown in FIG 6. The distal tip opening 314 may be offset from a central longitudinal axis X-X of the inner barrel 310, along with the plunger 330 may travel. In some instances, the distal tip opening 314 may be centered on a central longitudinal axis Y-Y of the outer barrel 320, as shown in FIG. 6. The space 321 between the inner barrel 310 and the outer barrel 320 may extend only partially around the inner barrel 310, as shown in FIG. 7. The outer barrel 320 may have a distal tubular extension 329 with an engagement member 328, and the inner barrel 310 may have an elongated distal tip 312 with a distal tip opening 314 extending distal of the distal end 322, and a plunger 330 disposed within the inner barrel 310. These structures may be similar to those described above for syringe 100, 200. As with the syringes 100, 200 described above, the chamber of the inner barrel 310 may be filled with liquid such as saline, and sealed with the plunger 330, such that the inner barrel 310 is devoid of air. The syringe 300 may be used in a similar manner as the syringes 100, 200 described above, with saline in the inner barrel 310 being expressed through the distal tip opening 314 under a liquid line in the connector, and any air being forced up through the space 321 between the inner barrel 310 and outer barrel 320. In some instances the upper or proximal end of the space 321 may be open to the ambient environment (e.g., atmosphere), or otherwise allow the air to escape from the connector through the lumen of the transverse extension of the connector while the syringe 300, namely the distal tubular extension 329 of the outer barrel 320, is sealingly coupled to the transverse extension of the connector. For example, the space 321 may be open to an ambient environment via a proximal opening of the outer barrel 320.

Currently, a wet-to-wet connection is used to de-air tubing connections in an ECLS system, but that process is prone to user error and is difficult for novice users. The syringe 100, 200, 300 is easy to use at any skill level. A method of de-airing a liquid conduit of an extracorporeal life support system may involve coupling any of the de-airing syringes 100, 200, 300 described above with a connector 10, 50 joining two sections of liquid-filled conduit. The inner barrel 110, 210, 310 may be filled with saline and sealed with the plunger 130, 330, and the space between the inner barrel 110, 210, 310 and the outer barrel 120, 220, 320 is empty. The outer barrel 120, 220, 320 of the syringe 100, 200, 300 may include an engagement member or coupling configured to couple with the connector 10, 50 of the liquid conduit. The distal tip 112, 212, 312 of the syringe 100, 200, 300 is inserted through the side lumen of the transverse extension of the connector and into the main lumen of the connector while the outer barrel 120, 220, 320 is sealingly connected to the transverse extension of the connector to create a fluid-tight seal. With the distal tip opening 114, 214, 314 positioned under a liquid line within the main lumen of the connector, the plunger 130, 330 is depressed to inject the saline inside the inner barrel into the main lumen of the connector thereby forcing air within the connector 10 upward along an exterior of the inner barrel 110, 210, 310 into the space 121, 221, 321 between the inner and outer barrels, as shown in FIGS. 4 and 5. Once all air within the connector 10 has been forced out of the lumen of the connector and into the exhaust space 121, 221, 321, and saline begins to enter the space 121, 221, 321 between the inner and outer barrels, the syringe 100, 200, 300 may be removed and the transverse extension 20 on the connector 10 may be capped, or otherwise closed, if desired.

While the above discussion describes an ECLS system, it is contemplated that the de-airing syringe, as described herein, may be utilized with any other system where an air-free connection between two sections of tubing is desired.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The scope of the disclosure is, of course, defined in the language in which the appended claims are expressed.

## Claims

1. A syringe for de-airing a liquid conduit of an extracorporeal life support system, the syringe comprising:
an inner barrel having an elongated distal tip defining a distal tip opening;
an outer barrel at least partially surrounding the inner barrel, an inner wall of the outer barrel spaced apart from at least a portion of an outer wall of the inner barrel defining a space therebetween, the outer barrel having a distal end with at least one opening, and a proximal opening, wherein the at least one opening is proximal of the distal tip opening of the inner barrel; and
a plunger disposed within the inner barrel and axially movable relative to the inner barrel, the plunger having a friction fit with an inner wall of the inner barrel.

2. The syringe of claim 1, wherein the inner barrel and the outer barrel are coaxial.

3. The syringe of claim 2, wherein one or more radially extending supports connect the outer wall of the inner barrel to the inner wall of the outer barrel.

4. The syringe of any one of the preceding claims, wherein the at least one opening in the outer barrel is a distally facing opening.

5. The syringe of claim 4, wherein the distally facing opening surrounds the elongated distal tip of the inner barrel.

6. The syringe of any one of the preceding claims, wherein the distal tip tapers distally to the distal tip opening.

7. The syringe of any one of the preceding claims, wherein the outer barrel includes a coupler for coupling with a connector in the liquid conduit of the extracorporeal life support system.

8. The syringe of claim 7, wherein the coupler includes a distal tubular extension with inner threading,

9. They syringe of claim 8, wherein the elongated distal tip of the inner barrel extends through the distal tubular extension and the distal tip opening is spaced apart distally from a distal end of the distal tubular extension.

10. The syringe of claim 8, wherein the distal tubular extension forms a luer lock coupling with the connector in the liquid conduit of the extracorporeal life support system.

11. The syringe of any one of the preceding claims, wherein the space is open to an ambient environment via the proximal opening.

12. The syringe of any one of the preceding claims, wherein a portion of the outer wall of the inner barrel is fixed to a portion of the inner wall of the outer barrel.

13. The syringe of claim 12, wherein the distal tip opening is offset from a central longitudinal axis of the inner barrel and centered on a central longitudinal axis of the outer barrel.

14. The syringe of any one of the preceding claims, wherein the inner barrel is filled with saline and sealed with the plunger.

15. They syringe of any one of the preceding claims, wherein the at least one opening is in fluid communication with the space between the inner barrel and the outer barrel.
